# EUROPEAN PATENT APPLICATION

(11) **EP 4 775 591 A1**
(43) Date of publication of application: **15.07.2026**
(21) Application number: 25874162.8
(22) Date of filing: 30.09.2025
(51) Int. Cl.: C07K 14/34, C12N 15/77, C12P 13/08, C12P 13/06, C12P 13/04

(54) **PROTEIN VARIANT AND METHOD FOR PRODUCING L-ISOLEUCINE BY USING SAME**

(30) Priority: 30.09.2024 KR 20240133210
(71) Applicant: CJ Cheiljedang Corporation, Seoul 04560 (KR)
(72) Inventor: YOON, Jeong-Hye, Seoul 04560 (KR); RYU, Si-Won, Seoul 04560 (KR); KIM, Bina, Seoul 04560 (KR)
(74) Representative: Meissner Bolte Partnerschaft mbB
(86) International application number: PCT/KR2025/015534
(87) International publication number: WO 2026/071848

(57) **Abstract**

The present disclosure relates to a microorganism of the genus *Corynebacterium* comprising a protein variant, a polynucleotide encoding the protein variant; or at least one selected from the group consisting of the protein variant and the polynucleotide; and a method for producing an L-amino acid using the microorganism.

## Description

### [Technical Field]

The present disclosure relates to a novel protein variant, a microorganism of the genus *Corynebacterium* comprising the protein variant, and a method for producing an L-amino acid using the microorganism.

### [Background Art]

In order to produce L-amino acids and other beneficial substances, various studies have been conducted to develop microorganisms with high-efficiency production and technologies for fermentation processes. For example, target-specific approaches, such as a method of increasing expression of a gene encoding an enzyme involved in L-threonine biosynthesis or a method of removing a gene unnecessary for biosynthesis, have been widely used (WO2008-013428 A1).

However, L-amino acids are in increasing demand, and accordingly, there still remains a need for further research on effectively increasing L-amino acid-producing ability.

### [Disclosure]

### [Technical Problem]

The problem to be solved by the present disclosure relates to a protein variant, comprising an amino acid sequence wherein, in the amino acid sequence of SEQ ID NO: 12, from the N-terminus, the amino acid corresponding to position 58 is substituted with arginine (R), the amino acid corresponding to position 352 is substituted with proline (P), the amino acid corresponding to position 433 is substituted with arginine (R), the amino acid corresponding to position 521 is substituted with arginine (R), the amino acid corresponding to position 710 is substituted with aspartic acid (D), or the amino acid corresponding to position 784 is substituted with cysteine (C); or a combination thereof; a polynucleotide encoding the protein variant; a microorganism of the genus *Corynebacterium,* comprising at least one selected from the group consisting of the protein variant and a polynucleotide encoding the protein variant; a method for producing an L-amino acid, comprising culturing the microorganism in a medium; and a use of the protein variant, polynucleotide, or microorganism for producing an L-amino acid.

### [Technical Solution]

An object of the present disclosure is to provide a novel protein variant.

Another object of the present disclosure is to provide a polynucleotide encoding the protein variant.

Still another object of the present disclosure is to provide a microorganism of the genus *Corynebacterium,* comprising at least one selected from the group consisting of the protein variant and a polynucleotide encoding the same.

Still another object of the present disclosure is to provide a method for producing an L-amino acid, comprising culturing the microorganism in a medium.

### [Advantageous Effects]

When a microorganism of the genus *Corynebacterium* comprising the novel protein variant of the present disclosure is cultured, it is possible to achieve high-yield production of L-amino acids compared to conventional microorganisms having unmodified polypeptides.

### [Detailed Description of Preferred Embodiments]

The present disclosure will be described in detail as follows. Meanwhile, each description and embodiment disclosed in the present disclosure may also be applied to other descriptions and embodiments. That is, all combinations of various elements disclosed in the present disclosure fall within the scope of the present disclosure. Further, the scope of the present disclosure is not limited by the specific description described below. In addition, a number of papers and patent documents are referenced and cited throughout the present specification. The disclosures of the cited papers and patent documents are incorporated herein by reference in their entirety to further clarify the level of art and the description of the present disclosure.

One aspect of the present disclosure provides a protein variant, comprising an amino acid sequence wherein, in the amino acid sequence of SEQ ID NO: 12, from the N-terminus, at least one amino acid selected from the group consisting of the amino acid corresponding to position 58, the amino acid corresponding to position 352, the amino acid corresponding to position 433, the amino acid corresponding to position 521, the amino acid corresponding to position 710, and the amino acid corresponding to position 784 are substituted with an amino acid different from the original amino acid.

In the present disclosure, the amino acid sequence of SEQ ID NO: 12 is as shown in Table 1 below.

**[Table 1]**

| Name of Protein (gene ID, gene name) | Sequence (N-Terminus → C-Terminus) | SEQ ID NO. |
|---|---|---|
| ThrA (Aspartokinase) | MRVLKFGGTSVANAERFLRVADILESNARQGQVATVLS APAKITNHLVAMIEKTISGQDALPNISDAERIFAELLTGLA AAQPGFPLAQLKTFVDQEFAQIKHVLHGISLLGQCPDSI | 12 |
| | NAALICRGEKMSIAIMAGVLEARGHNVTVIDPVEKLLAV GHYLESTVDIAESTRRIAASRIPADHMVLMAGFTAGNEK GELVVLGRNGSDYSAAVLAACLRADCCEIWTDVDGVYT CDPRQVPDARLLKSMSYQEAMELSYFGAKVLHPRTITPI AQFQIPCLIKNTGNPQAPGTLIGASRDEDELPVKGISNLN NMAMFSVSGPGMKGMVGMAARVFAAMSRARISVVLIT QSSSEYSISFCVPQSDCVRAERAMQEEFYLELKEGLLE PLAVTERLAIISVVGDGMRTLRGISAKFFAALARANINIVA IAQGSSERSISVVVNNDDATTGVRVTHQMLFNTDQVIEV FVIGVGGVGGALLEQLKRQQSWLKNKHIDLRVCGVANS KALLTNVHGLNLENWQEELAQAKEPFNLGRLIRLVKEY HLLNPVIVDCTSSQAVADQYADFLREGFHVVTPNKKAN TSSMDYYHLLRHAAEKSRRKFLYDTNVGAGLPVIENLQ NLLNAGDELMKFSGILSGSLSYIFGKLDEGMSFSEATTL AREMGYTEPDPRDDLSGMDVARKLLILARETGRELELA DIEIEPVLPAEFNAEGDVAAFMANLSQLDDLFAARVAKA RDEGKVLRYVGNIDEDGACRVKIAEVDGNDPLFKVKNG ENALAFYSHYYQPLPLVLRGYGAGNDVTAAGVFADLLR TLSWKLGV. | |

In one embodiment, the protein variant of the present disclosure described above may essentially consist of an amino acid sequence wherein, in the amino acid sequence of SEQ ID NO: 12, from the N-terminus, the amino acid corresponding to position 352, the amino acid corresponding to position 433, and the amino acid corresponding to position 521 are substituted with an amino acid different from the original amino acid.

In another embodiment, the protein variant of the present disclosure described above may consist of an amino acid sequence wherein, in the amino acid sequence of SEQ ID NO: 12, from the N-terminus, at least one amino acid selected from the group consisting of the amino acid corresponding to position 352, the amino acid corresponding to position 433, and the amino acid corresponding to position 521 is substituted with an amino acid different from the original amino acid.

In another embodiment, the protein variant of the present disclosure described above may consist of an amino acid sequence wherein, in the amino acid sequence of SEQ ID NO: 12, from the N-terminus, at least one amino acid selected from the group consisting of the amino acid corresponding to position 58, the amino acid corresponding to position 352, the amino acid corresponding to position 433, the amino acid corresponding to position 521, and the amino acid corresponding to position 710 is substituted with an amino acid different from the original amino acid.

In another embodiment, the protein variant of the present disclosure described above may consist of an amino acid sequence wherein, in the amino acid sequence of SEQ ID NO: 12, from the N-terminus, at least one amino acid selected from the group consisting of the amino acid corresponding to position 352, the amino acid corresponding to position 433, the amino acid corresponding to position 521, and the amino acid corresponding to position 710 is substituted with an amino acid different from the original amino acid.

In another embodiment, the protein variant of the present disclosure described above may consist of an amino acid sequence wherein, in the amino acid sequence of SEQ ID NO: 12, from the N-terminus, at least one amino acid selected from the group consisting of the amino acid corresponding to position 58, the amino acid corresponding to position 352, the amino acid corresponding to position 433, the amino acid corresponding to position 521, the amino acid corresponding to position 710, and the amino acid corresponding to position 784 is substituted with an amino acid different from the original amino acid.

In the present disclosure, the amino acid sequence of SEQ ID NO: 12 may be a sequence of a ThrA (Aspartokinase) protein. In one embodiment, the protein may be a protein encoded by the *thrA* gene.

In one embodiment, the amino acid corresponding to position 58 from the N-terminus in the amino acid sequence of SEQ ID NO: 12 may be glutamine (Gln, Q), but is not limited thereto.

In another embodiment, the amino acid corresponding to position 352 from the N-terminus in the amino acid sequence of SEQ ID NO: 12 may be serine (Ser, S), but is not limited thereto.

In another embodiment, the amino acid corresponding to position 433 from the N-terminus in the amino acid sequence of SEQ ID NO: 12 may be glycine (Gly, G), but is not limited thereto.

In another embodiment, the amino acid corresponding to position 521 from the N-terminus in the amino acid sequence of SEQ ID NO: 12 may be tryptophan (Trp, W), but is not limited thereto.

In another embodiment, the amino acid corresponding to position 710 from the N-terminus in the amino acid sequence of SEQ ID NO: 12 may be asparagine (Asn, N), but is not limited thereto.

In another embodiment, the amino acid corresponding to position 784 from the N-terminus in the amino acid sequence of SEQ ID NO: 12 may be tyrosine (Tyr, Y), but is not limited thereto.

In one embodiment, the protein variant of the present disclosure may comprise an amino acid sequence wherein, from the N-terminus, the amino acid corresponding to position 58 is substituted with arginine (R), the amino acid corresponding to position 352 is substituted with proline (P), the amino acid corresponding to position 433 is substituted with arginine (R), the amino acid corresponding to position 521 is substituted with arginine (R), the amino acid corresponding to position 710 is substituted with aspartic acid (D), the amino acid corresponding to position 784 is substituted with cysteine (C), or a combination thereof.

In another embodiment, the protein variant of the present disclosure may comprise an amino acid sequence wherein, from the N-terminus, the amino acid corresponding to position 352 is substituted with proline (P), the amino acid corresponding to position 433 is substituted with arginine (R), and the amino acid corresponding to position 521 is substituted with arginine (R).

In another embodiment, the protein variant of the present disclosure may comprise an amino acid sequence wherein, from the N-terminus, the amino acid corresponding to position 58 is substituted with arginine (R), the amino acid corresponding to position 352 is substituted with proline (P), the amino acid corresponding to position 433 is substituted with arginine (R), the amino acid corresponding to position 521 is substituted with arginine (R), and the amino acid corresponding to position 710 is substituted with aspartic acid (D).

In another embodiment, the protein variant of the present disclosure may comprise an amino acid sequence wherein, from the N-terminus, the amino acid corresponding to position 352 is substituted with proline (P), the amino acid corresponding to position 433 is substituted with arginine (R), the amino acid corresponding to position 521 is substituted with arginine (R), and the amino acid corresponding to position 710 is substituted with aspartic acid (D).

In another embodiment, the protein variant of the present disclosure may comprise an amino acid sequence wherein, from the N-terminus, the amino acid corresponding to position 58 is substituted with arginine (R), the amino acid corresponding to position 352 is substituted with proline (P), the amino acid corresponding to position 433 is substituted with arginine (R), the amino acid corresponding to position 521 is substituted with arginine (R), the amino acid corresponding to position 710 is substituted with aspartic acid (D), and the amino acid corresponding to position 784 is substituted with cysteine (C).

The protein variant of the present disclosure may have an activity of increasing L-amino acid-producing ability compared to a wild-type protein (polypeptide).

The protein variant of the present disclosure may comprise an amino acid sequence having homology or identity of at least 70%, 75%, 80%, 85%, 90%, 95%, 96%, 97%, 98%, 99%, 99.5%, or 99.7% to: an amino acid sequence wherein, in the amino acid sequence of SEQ ID NO: 12, from the N-terminus, the amino acids corresponding to positions 352, 433, and 521 are substituted with an amino acid different from the original amino acid; an amino acid sequence wherein, in the amino acid sequence of SEQ ID NO: 12, from the N-terminus, the amino acids corresponding to positions 58, 352, 433, 521, and 710 are substituted with an amino acid different from the original amino acid; an amino acid sequence wherein, in the amino acid sequence of SEQ ID NO: 12, from the N-terminus, the amino acids corresponding to positions 352, 433, 521, and 710 are substituted with an amino acid different from the original amino acid; or an amino acid sequence wherein, in the amino acid sequence of SEQ ID NO: 12, from the N-terminus, the amino acids corresponding to positions 58, 352, 433, 521, 710, and 784 are substituted with an amino acid different from the original amino acid. Additionally, it is apparent that any variant having an amino acid sequence, in which a part of the sequence has a deletion, modification, substitution, conservative substitution, or addition, also falls within the scope of the present disclosure as long as the amino acid sequence has such homology or identity and exhibits an efficacy corresponding to that of the variant of the present disclosure (efficacy of increasing the L-amino acid-producing ability).

For example, this includes cases of having addition or deletion of a sequence that does not alter the function of the variant of the present disclosure, naturally occurring mutation, silent mutation, or conservative substitution at the N-terminus, the C-terminus, and/or within the amino acid sequence.

As used herein, the term "conservative substitution" refers to a substitution of one amino acid with another amino acid having similar structural and/or chemical properties. Such an amino acid substitution may generally occur based on similarity in the polarity, charge, solubility, hydrophobicity, hydrophilicity, and/or amphipathic nature of residues. Typically, conservative substitutions may have little or no effect on the activity of the protein or polypeptide.

As used herein, the term "variant" refers to a polypeptide having at least one amino acid different from the amino acid sequence of the variant before mutation by conservative substitution and/or modification such that the functions and properties of the protein are retained. Such a variant may generally be identified by modifying at least one amino acid in the amino acid sequence of the polypeptide and evaluating the properties of the modified polypeptide. That is, the ability of the variant may be enhanced, unchanged, or reduced relative to a polypeptide before mutation. Additionally, some variants may include variants in which at least one portion, such as an N-terminal leader sequence or transmembrane domain, have been removed. Other variants may include variants in which a region has been partially removed from the N- and/or C-terminal of a mature protein. The term "variant" may be used interchangeably with terms such as modification, modified polypeptide, modified protein, mutant, mutein, divergent, etc., but is not limited thereto, as long as the terms are used to indicate mutation.

Additionally, the variant may also comprise deletion or addition of amino acids that have minimal influence on the properties and secondary structure of a polypeptide. For example, a signal (or leader) sequence involved in the co-translational or post-translational translocation of proteins may be conjugated to the N-terminus of the variant. Further, the variant may also be conjugated with another sequence or linker for identification, purification, or synthesis.

In one embodiment, the protein variant of the present disclosure described above may have homology or identity of at least 90%, 91%, 93%, 94%, 95%, 96%, 97%, 98%, or 99%, and less than 100% to the amino acid sequence of SEQ ID NO: 12. That is, the protein variant of the present disclosure described above may have an amino acid sequence identity of at least 90%, 91%, 93%, 94%, 95%, 96%, 97%, 98%, or 99%, and less than 100%, respectively, to the amino acid sequence of SEQ ID NO: 12.

Another aspect of the present disclosure provides a protein variant comprising any one amino acid sequence selected from the group consisting of the amino acid sequences of SEQ ID NO: 15 to SEQ ID NO: 18. In one embodiment, the protein variant may be a protein variant comprising the amino acid sequence of SEQ ID NO: 15, SEQ ID NO: 16, SEQ ID NO: 17, or SEQ ID NO: 18. In another embodiment, the variant of the present disclosure may essentially consist of the amino acid sequence of SEQ ID NO: 15, SEQ ID NO: 16, SEQ ID NO: 17, or SEQ ID NO: 18. In still another embodiment, the variant of the present disclosure may consist of the amino acid sequence of SEQ ID NO: 15, SEQ ID NO: 16, SEQ ID NO: 17, or SEQ ID NO: 18.

In the present disclosure, the amino acid sequence of SEQ ID NO: 15 may be a sequence of a protein variant wherein, in the protein (ThrA) encoded by the *thrA* gene, the amino acid corresponding to position 352 is substituted with proline (P), the amino acid corresponding to position 433 is substituted with arginine (R), and the amino acid corresponding to position 521 is substituted with arginine (R).

In the present disclosure, the amino acid sequence of SEQ ID NO: 16 may be a sequence of a protein variant wherein, in the protein (ThrA) encoded by the *thrA* gene, the amino acid corresponding to position 58 is substituted with arginine (R), the amino acid corresponding to position 352 is substituted with proline (P), the amino acid corresponding to position 433 is substituted with arginine (R), the amino acid corresponding to position 521 is substituted with arginine (R), and the amino acid corresponding to position 710 is substituted with aspartic acid (D).

In the present disclosure, the amino acid sequence of SEQ ID NO: 17 may be a sequence of a protein variant wherein, in the protein (ThrA) encoded by the *thrA* gene, the amino acid corresponding to position 352 is substituted with proline (P), the amino acid corresponding to position 433 is substituted with arginine (R), the amino acid corresponding to position 521 is substituted with arginine (R), and the amino acid corresponding to position 710 is substituted with aspartic acid (D).

In the present disclosure, the amino acid sequence of SEQ ID NO: 18 may be a sequence of a protein variant wherein, in the protein (ThrA) encoded by the *thrA* gene, the amino acid corresponding to position 58 is substituted with arginine (R), the amino acid corresponding to position 352 is substituted with proline (P), the amino acid corresponding to position 433 is substituted with arginine (R), the amino acid corresponding to position 521 is substituted with arginine (R), the amino acid corresponding to position 710 is substituted with aspartic acid (D), and the amino acid corresponding to position 784 is substituted with cysteine (C).

The protein variant of the present disclosure may comprise an amino acid sequence selected from the group consisting of the amino acid sequences of SEQ ID NO: 15 to SEQ ID NO: 18 (such as the amino acid sequence of SEQ ID NO: 15, SEQ ID NO: 16, SEQ ID NO: 17, or SEQ ID NO: 18), or an amino acid sequence having homology or identity of at least 70%, 75%, 80%, 85%, 90%, 95%, 96%, 97%, 98%, 99%, 99.5%, 99.7%, or 99.9% thereto. Additionally, it is apparent that any variant having an amino acid sequence, in which a part of the sequence has a deletion, modification, substitution, conservative substitution, or addition, also falls within the scope of the present disclosure, as long as the amino acid sequence has such homology or identity and exhibits an efficacy corresponding to that of the variant of the present disclosure (efficacy of increasing L-amino acid production).

For example, this includes cases of having addition or deletion of a sequence that does not alter the function of the variant of the present disclosure, naturally occurring mutation, silent mutation, or conservative substitution at the N-terminus, the C-terminus, and/or within the amino acid sequence.

With respect to the protein variant, conservative substitution, variant, *etc.* are as described above.

As used herein, the term "homology" or "identity" refers to a degree of similarity between two given amino acid sequences or nucleotide sequences, and may be expressed as a percentage. The terms homology and identity may often be used interchangeably.

The sequence homology or identity of a conserved polynucleotide or polypeptide is determined by standard alignment algorithms, and a default gap penalty established by the program used may be used together. Substantially, homologous or identical sequences are generally capable of hybridizing with the whole sequence or a part of the sequence under moderately or highly stringent conditions. It is apparent that hybridization also includes hybridization with a polynucleotide containing a general codon or a codon considering codon degeneracy in the polynucleotide.

Whether any two polynucleotide or polypeptide sequences have homology, similarity, or identity may be determined by, for example, a known computer algorithm such as the "FASTA" program using default parameters as in Pearson et al. (1988) [Proc. Natl. Acad. Sci. USA 85]: 2444. Alternatively, they may be determined using the Needleman-Wunsch algorithm (Needleman and Wunsch, 1970, J. Mol. Biol. 48: 443-453) as performed in the Needleman program of the EMBOSS package (EMBOSS: The European Molecular Biology Open Software Suite, Rice et al., 2000, Trends Genet. 16: 276-277) (version 5.0.0 or later) (including GCG program package (Devereux, J., et al, Nucleic Acids Research 12: 387 (1984)), BLASTP, BLASTN, and FASTA (Atschul, [S.] [F.,] [ET AL, J MOLEC BIOL 215]: 403 (1990); Guide to Huge Computers, Martin J. Bishop, [ED.,] Academic Press, San Diego, 1994, and [CARILLO ET AL.] (1988) SIAM J Applied Math 48: 1073)). For example, homology, similarity, or identity may be determined using BLAST of the National Center for Biotechnology Information, or ClustalW.

The homology, similarity, or identity between polynucleotides or polypeptides may be determined by, for example, comparing sequence information using a GAP computer program, such as Needleman et al., (1970), J Mol Biol. 48:443, as described in, for example, Smith and Waterman, Adv. Appl. Math (1981) 2:482. Briefly, a GAP program may be defined as the value acquired by dividing the number of similarly aligned symbols (namely, nucleotides or amino acids) by the total number of symbols in the shorter of two sequences. The default parameters for the GAP program may comprise: (1) a binary comparison matrix (comprising values of 1 for identity and 0 for non-identity) and a weighted comparison matrix of Gribskov et al. (1986) Nucl. Acids Res. 14: 6745 (or EDNAFULL (EMBOSS version of NCBI NUC4.4) substitution matrix), as disclosed in Schwartz and Dayhoff, eds., Atlas Of Protein Sequence And Structure, National Biomedical Research Foundation, pp. 353-358 (1979); (2) a penalty of 3.0 for each gap and an additional 0.10 penalty for each symbol in each gap (or gap opening penalty of 10, gap extension penalty of 0.5); and (3) no penalty for end gaps.

In one example of the present disclosure, the variant of the present disclosure described above may have an activity of increasing L-amino acid-producing ability compared to a wild-type protein (polypeptide).

The L-amino acid may be selected from the group consisting of L-threonine, L-isoleucine, O-acetyl L-homoserine, and L-glycine, but is not limited thereto.

As used herein, the term "corresponding to" refers to an amino acid residue at the position recited in a peptide, or an amino acid residue which is similar, identical, or homologous to the residue recited in a peptide. Identifying an amino acid at a corresponding position may be determining a particular amino acid in a sequence that refers to a particular sequence. As used herein, the term "corresponding region" generally refers to a similar or corresponding position in a related protein or reference protein.

For example, any amino acid sequence can be aligned with an amino acid sequence wherein, in the amino acid sequence of SEQ ID NO: 12, from the N-terminus, the amino acids corresponding to positions 352, 433, and 521 are substituted with an amino acid different from the original amino acid,
an amino acid sequence wherein, in the amino acid sequence of SEQ ID NO: 12, from the N-terminus, the amino acids corresponding to positions 58, 352, 433, 521, and 710 are substituted with an amino acid different from the original amino acid,
an amino acid sequence wherein, in the amino acid sequence of SEQ ID NO: 12, from the N-terminus, the amino acids corresponding to positions 352, 433, 521, and 710 are substituted with an amino acid different from the original amino acid,
an amino acid sequence wherein, in the amino acid sequence of SEQ ID NO: 12, from the N-terminus, the amino acids corresponding to positions 58, 352, 433, 521, 710, and 784 are substituted with an amino acid different from the original amino acid, or
any one amino acid sequence selected from the group consisting of the amino acid sequences of SEQ ID NO: 15 to SEQ ID NO: 18 (such as the amino acid sequence of SEQ ID NO: 15, SEQ ID NO: 16, SEQ ID NO: 17, or SEQ ID NO: 18);
and based on the alignment, each amino acid residue of the amino acid sequence can be numbered with reference to the numerical position of the amino acid residue corresponding to the amino acid residue of an amino acid sequence wherein, in the amino acid sequence of SEQ ID NO: 12, from the N-terminus, the amino acids corresponding to positions 352, 433, and 521 are substituted with an amino acid different from the original amino acid,
an amino acid sequence wherein, in the amino acid sequence of SEQ ID NO: 12, from the N-terminus, the amino acids corresponding to positions 58, 352, 433, 521, and 710 are substituted with an amino acid different from the original amino acid,
an amino acid sequence wherein, in the amino acid sequence of SEQ ID NO: 12, from the N-terminus, the amino acids corresponding to positions 352, 433, 521, and 710 are substituted with an amino acid different from the original amino acid,
an amino acid sequence wherein, in the amino acid sequence of SEQ ID NO: 12, from the N-terminus, the amino acids corresponding to positions 58, 352, 433, 521, 710, and 784 are substituted with an amino acid different from the original amino acid, or
any one amino acid sequence selected from the group consisting of the amino acid sequences of SEQ ID NO: 15 to SEQ ID NO: 18 (such as the amino acid sequence of SEQ ID NO: 15, SEQ ID NO: 16, SEQ ID NO: 17, or SEQ ID NO: 18).

For example, a sequence alignment algorithm such as that described herein can identify the position of an amino acid or a position where modifications such as substitutions, insertions or deletions occur as compared to a query sequence (also referred to as a "reference sequence").

For such alignments, for example, the Needleman-Wunsch algorithm (Needleman and Wunsch, 1970, J. Mol. Biol. 48: 443-453), the Needle program of the EMBOSS package (EMBOSS: The European Molecular Biology Open Software Suite, Rice et al., 2000, Trends Genet. 16: 276-277), *etc.* may be used, but are not limited thereto, and sequence alignment programs, pairwise sequence comparison algorithms, *etc.,* known in the art may be appropriately used.

Another aspect of the present disclosure provides a polynucleotide encoding the protein variant of the present disclosure.

As used herein, the term "polynucleotide" refers to a DNA or RNA strand having at least a certain length, which is a polymer of nucleotides in which nucleotide monomers are connected in a long chain by covalent bonds. More specifically, it refers to a polynucleotide fragment encoding the variant.

In one example, the polynucleotide may be a polynucleotide encoding a protein variant comprising: an amino acid sequence wherein, in the amino acid sequence of SEQ ID NO: 12, from the N-terminus, the amino acid corresponding to position 352, the amino acid corresponding to position 433, and the amino acid corresponding to position 521 are substituted with an amino acid different from the original amino acid;
an amino acid sequence wherein, in the amino acid sequence of SEQ ID NO: 12, from the N-terminus, at least one amino acid selected from the group consisting of the amino acid corresponding to position 58, the amino acid corresponding to position 352, the amino acid corresponding to position 433, the amino acid corresponding to position 521, and the amino acid corresponding to position 710 is substituted with an amino acid different from the original amino acid;
an amino acid sequence wherein, in the amino acid sequence of SEQ ID NO: 12, from the N-terminus, at least one amino acid selected from the group consisting of the amino acid corresponding to position 352, the amino acid corresponding to position 433, the amino acid corresponding to position 521, and the amino acid corresponding to position 710 is substituted with an amino acid different from the original amino acid; or
an amino acid sequence wherein, in the amino acid sequence of SEQ ID NO: 12, from the N-terminus, at least one amino acid selected from the group consisting of the amino acid corresponding to position 58, the amino acid corresponding to position 352, the amino acid corresponding to position 433, the amino acid corresponding to position 521, the amino acid corresponding to position 710, and the amino acid corresponding to position 784 is substituted with an amino acid different from the original amino acid.

In one example, the polynucleotide may be a polynucleotide comprising any one nucleotide sequence selected from the group consisting of the nucleotide sequences of SEQ ID NO: 22 to SEQ ID NO: 25, and for example, may comprise the nucleotide sequences of SEQ ID NO: 22 to SEQ ID NO: 25.

The polynucleotide encoding the protein variant of the present disclosure may have or comprise a nucleotide sequence selected from the group consisting of the nucleotide sequences of SEQ ID NO: 22 to SEQ ID NO: 25, for example, the nucleotide sequence of SEQ ID NO: 22, SEQ ID NO: 23, SEQ ID NO: 24, or SEQ ID NO: 25. In another example, the polynucleotide may comprise a nucleotide sequence having homology or identity of at least 70%, 75%, 80%, 85%, 90%, 95%, 96%, 97%, 98%, 99%, 99.5%, 99.7%, or 99.9% to the nucleotide sequences above. Additionally, it is apparent that any polynucleotide having a nucleotide sequence, in which a part of the sequence has a deletion, modification, substitution, conservative substitution, or addition, also falls within the scope of the present disclosure, as long as the sequence encoding a polypeptide or a protein has such homology or identity and exhibits an efficacy corresponding to that of the variant of the present disclosure (efficacy of increasing L-amino acid production).

In one embodiment, the nucleotide sequence or amino acid sequence provided in the present specification may comprise one modified by common mutagenesis, for example, directed evolution and/or site-directed mutagenesis, *etc.,* within a range that maintains their original functions or target functions.

The polynucleotide of the present disclosure may have various modifications in coding regions within a range that does not change the amino acid sequence of the variant of the present disclosure, in consideration of degeneracy of codons or codons preferred in an organism in which the variant of the present disclosure is to be expressed. Specifically, the polynucleotide of the present disclosure may have or comprise a nucleotide sequence with homology or identity of at least 70%, at least 75%, at least 80%, at least 85%, at least 90%, at least 95%, at least 96%, at least 97%, at least 98%, and less than 100% to any one nucleotide sequence selected from the group consisting of the nucleotide sequences of SEQ ID NO: 22 to SEQ ID NO: 25 (for example, the nucleotide sequence of SEQ ID NO: 22, SEQ ID NO: 23, SEQ ID NO: 24, or SEQ ID NO: 25), or consist of or essentially consist of a nucleotide sequence having homology or identity of at least 70%, at least 75%, at least 80%, at least 85%, at least 90%, at least 95%, at least 96%, at least 97%, at least 98%, and less than 100% to the nucleotide sequences above, but is not limited thereto.

In addition, the polynucleotide of the present disclosure may comprise a probe that can be prepared from a known gene sequence without limitation, for example, any sequence capable of hybridizing with a complementary sequence to all or a part of the polynucleotide sequence of the present disclosure under stringent conditions. The "stringent condition" refers to a condition that allows specific hybridization between polynucleotides. For example, the stringent conditions may include conditions under which polynucleotides having a high homology or identity of at least 70%, at least 75%, at least 80%, at least 85%, at least 90%, at least 95%, at least 96%, at least 97%, at least 98%, or at least 99% are hybridized with each other and polynucleotides having a homology or identity lower than the above are not hybridized with each other; or common washing conditions of Southern hybridization, that is, washing once, specifically, twice or three times at a salt concentration and a temperature corresponding to 60°C, 1 × SSC, 0.1% SDS, specifically, 60°C, 0.1 × SSC, 0.1% SDS, and more specifically 68°C, 0.1 × SSC, 0.1% SDS.

Hybridization requires that two nucleic acids have complementary sequences, although mismatches between bases may be possible depending on the stringency of hybridization. The term "complementary" is used to describe the relationship between nucleotide bases capable of hybridizing with each other. For example, with regard to DNA, adenine is complementary to thymine, and cytosine is complementary to guanine. Accordingly, the polynucleotide of the present disclosure may also comprise substantially similar nucleic acid sequences as well as isolated nucleic acid fragments that are complementary to the entire sequence.

Specifically, polynucleotides having a homology or identity with the polynucleotide of the present disclosure use hybridization conditions comprising hybridization at a Tₘ value of 55°C, and may be detected under the above conditions. Further, the Tₘ value may be 60°C, 63°C, or 65°C, but is not limited thereto, and may be appropriately adjusted by those skilled in the art depending on the purpose thereof.

The appropriate stringency for hybridizing the polynucleotides depends on the length of the polynucleotides and the degree of complementation, and the variables are well known in the art.

Still another aspect of the present disclosure is to provide a vector comprising the polynucleotide of the present disclosure. The vector may be an expression vector for expressing the polynucleotide in a host cell, but is not limited thereto.

The vector of the present disclosure may comprise a DNA construct comprising the nucleotide sequence of a polynucleotide encoding the target polypeptide operably linked to a suitable expression regulatory region (or expression regulatory sequence) so as to enable expression of the target polypeptide in a suitable host cell. The expression regulatory region may comprise a promoter capable of initiating transcription, any operator sequence for controlling such transcription, a sequence encoding a suitable mRNA ribosome binding site, and a sequence controlling termination of transcription and translation. The vector, after being transformed into a suitable host cell, may replicate or function independently of the host genome, or may be integrated into the genome itself.

The vector used in the present disclosure is not particularly limited, and any vector known in the art may be used. Examples of commonly used vectors comprise natural or recombinant plasmids, cosmids, viruses, and bacteriophages. For example, pWE15, M13, MBL3, MBL4, IXII, ASHII, APII, t10, t11, Charon4A, Charon21A, *etc.* may be used as a phage vector or a cosmid vector, and pDZ system, pBR system, pUC system, pBluescript II system, pGEM system, pTZ system, pCL system, pET system, etc. may be used as a plasmid vector. Specifically, pDZ, pDC, pDCM2, pACYC177, pACYC184, pCL, pECCG117, pUC19, pBR322, pMW118, pCC1BAC, pDC24 vectors, *etc.* may be used.

In one example, a polynucleotide encoding a target polypeptide may be inserted into the chromosome through a vector for intracellular chromosomal insertion. The insertion of the polynucleotide into the chromosome may be performed by any method known in the art, for example, by homologous recombination, but is not limited thereto. The vector may further comprise a selection marker to confirm the insertion into the chromosome. The selection marker is for selecting the cells transformed with the vector, that is, for confirming whether the target nucleic acid molecule has been inserted, and markers that provide selectable phenotypes, such as drug resistance, auxotrophy, resistance to cell toxic agents, or expression of surface polypeptides, may be used. Only cells expressing the selection marker are able to survive or to show different phenotypes under the environment treated with the selective agent, and thus the transformed cells may be selected.

As used herein, the term "transformation" refers to the introduction of a vector comprising a polynucleotide encoding a target polypeptide into a host cell or a microorganism such that the polypeptide encoded by the polynucleotide can be expressed in the host cell. The transformed polynucleotide may be inserted and located within a chromosome of the host cell or located outside the chromosome, and both cases can be included as long as it can be expressed in the host cell. Further, the polynucleotide comprises a DNA and/or RNA encoding the target polypeptide. The polynucleotide may be introduced in any form, as long as it can be introduced into the host cell and expressed therein. For example, the polynucleotide may be introduced into the host cell in the form of an expression cassette, which is a gene construct comprising all elements required for self-expression. The expression cassette may commonly comprise a promoter operably linked to the polynucleotide, a transcription termination signal, a ribosome binding site, and a translation termination signal. The expression cassette may be in the form of a self-replicable expression vector. Additionally, the polynucleotide may be introduced into a host cell as it is and operably linked to sequences required for expression in the host cell, but is not limited thereto.

Further, as used herein, the term "operably linked" means that the polynucleotide sequence is functionally linked to a promoter sequence that initiates and mediates transcription of the polynucleotide encoding the target variant of the present disclosure.

Still another aspect of the present disclosure provides a microorganism of the genus *Corynebacterium,* comprising at least one selected from the group consisting of the protein variant of the present disclosure and the polynucleotide of the present disclosure.

The microorganism of the present disclosure may comprise the protein variant of the present disclosure, a polynucleotide encoding the protein variant, or a vector comprising the polynucleotide of the present disclosure.

As used herein, the term "microorganism (or strain)" comprises all wild-type microorganisms or naturally or artificially genetically modified microorganisms, may be a microorganism in which a particular mechanism is weakened or enhanced due to insertion of a foreign gene, enhancement or inactivation of the activity of an endogenous gene, *etc.,* and may be a microorganism comprising genetic modification to produce a target polypeptide, protein, or product.

The microorganism of the present disclosure may be a microorganism comprising at least one of the protein variant of the present disclosure, the polynucleotide of the present disclosure, and the vector comprising the polynucleotide of the present disclosure; a microorganism modified to express the variant of the present disclosure or the polynucleotide of the present disclosure; a microorganism (*e.g.,* a recombinant microorganism) expressing the variant of the present disclosure or the polynucleotide of the present disclosure; or a microorganism (e.g., a recombinant microorganism) having an activity of the variant of the present disclosure, but is not limited thereto.

The microorganism of the present disclosure may have an L-amino acid-producing ability.

The microorganism of the present disclosure may have an increased L-amino acid-producing ability compared to a microorganism of the genus *Corynebacterium* not comprising at least one selected from the group consisting of the protein variant of the present disclosure and a polynucleotide encoding the protein variant described above (that is, a microorganism that does not express a protein variant comprising an amino acid sequence wherein, in the amino acid sequence of SEQ **ID** NO: 12, from the N-terminus, the amino acid corresponding to position 58 is substituted with arginine (R), the amino acid corresponding to position 352 is substituted with proline (P), the amino acid corresponding to position 433 is substituted with arginine (R), the amino acid corresponding to position 521 is substituted with arginine (R), the amino acid corresponding to position 710 is substituted with aspartic acid (D), or the amino acid corresponding to position 784 is substituted with cysteine (C), or a combination thereof; a microorganism that does not express a protein variant comprising any one amino acid sequence selected from the group consisting of the amino acid sequences of SEQ ID NO: 15 to SEQ ID NO: 18, for example, an amino acid sequence of SEQ ID NO: 15, SEQ ID NO: 16, SEQ ID NO: 17, or SEQ ID NO: 18; or a microorganism that expresses a wild-type protein corresponding to the protein variant).

The microorganism of the present disclosure may be a microorganism that naturally has an L-amino acid-producing ability, or a microorganism wherein a parent strain that does not naturally have a L-amino acid-producing ability has been introduced with the variant of the present disclosure or the polynucleotide encoding the same (or a vector comprising the polynucleotide), and/or a microorganism imparted with an L-amino acid-producing ability, but is not limited thereto.

In one example, the microorganism of the present disclosure is a cell or microorganism transformed with the polynucleotide of the present disclosure or a vector comprising a polynucleotide encoding the variant of the present disclosure to express the protein variant of the present disclosure, and for the purposes of the present disclosure, the strain of the present disclosure may comprise all microorganisms capable of producing an L-amino acid by including the variant of the present disclosure. For example, the microorganism of the present disclosure may be a recombinant microorganism whose L-amino acid-producing ability is increased by introducing a polynucleotide encoding the variant of the present disclosure into a natural wild-type microorganism or a microorganism producing an L-amino acid.

In one specific embodiment, the microorganism of the present disclosure may have an increased L-amino acid-producing ability compared to a microorganism of the genus *Corynebacterium* not comprising at least one selected from the group consisting of the protein variant of the present disclosure and a polynucleotide encoding the protein variant. The microorganism of the genus *Corynebacterium* not comprising at least one selected from the group consisting of the protein variant of the present disclosure and a polynucleotide encoding the protein variant may be a natural wild-type microorganism or a non-modified microorganism, and may also be expressed as a parent strain.

In the present disclosure, a microorganism (non-modified microorganism) as a host cell may be (1) a microorganism naturally having an L-amino acid-producing ability, (2) a microorganism naturally lacking or having a significantly low L-amino acid-producing ability, and/or (3) a microorganism imparted with an L-amino acid-producing ability or an enhanced L-amino acid-producing ability by introduction (transformation) of a mutation into the microorganism naturally having an L-amino acid-producing ability or the microorganism naturally lacking or having a significantly low L-amino acid-producing ability. In one specific embodiment, the microorganism as a host cell may be *Corynebacterium glutamicum* ATCC13032 strain, *Corynebacterium glutamicum* KCCM12502P (Korean Patent No. 10-2126951), *Corynebacterium glutamicum* KCCM12739P (Korean Patent No. 10-2363913), or *Corynebacterium glutamicum* KCCM12634P (Korean Patent No. 10-2182497), but is not limited thereto.

In one specific embodiment, the microorganism (variant microorganism or microorganism as a host cell) may be a microorganism in which an L-amino acid biosynthetic pathway has been further enhanced to increase L-amino acid production, but is not limited thereto.

In one example, the microorganism may be a microorganism in which feedback inhibition of L-threonine dehydratase has been released, feedback inhibition of homoserine dehydrogenase has been released, and/or feedback inhibition of aspartate kinase has been released.

In one specific embodiment, the microorganism may be a microorganism in which feedback inhibition of L-threonine dehydratase has been released.

As used herein, the term "threonine dehydratase (EC 4.3.1.19)" refers to an enzyme that produces 2-ketobutyrate from threonine, is encoded by the *ilvA* gene, and is known to be subject to feedback inhibition by L-isoleucine. Microorganisms of the genus *Corynebacterium* synthesize L-isoleucine through three intermediate metabolites using pyruvate and 2-ketobutyrate as precursors. The amino acid sequence of the threonine dehydratase can be obtained from publicly known databases such as NCBI GenBank or from US 2023-0098971 A1 and US 10982244 B2.

In another specific embodiment, the microorganism may be a microorganism in which feedback inhibition of homoserine dehydrogenase has been released.

As used herein, the term "homoserine dehydrogenase (EC:1.1.1.3)" refers to an enzyme that is encoded by the *hom* gene and catalyzes the synthesis of homoserine. Homoserine dehydrogenase is known to be subject to feedback inhibition by isoleucine. The amino acid sequence of the homoserine dehydrogenase can be obtained from publicly known databases such as NCBI GenBank or from US 10982244 B2.

In one specific embodiment, the microorganism may be a microorganism in which feedback inhibition of aspartate kinase has been released.

As used herein, the term "aspartate kinase (EC: 2.7.2.4)" refers to an enzyme that is encoded by the *lysC* gene, and the amino acid sequence of the aspartate kinase can be obtained from publicly known databases such as NCBI GenBank or from US 10662450 B2.

As used herein, the term "release of feedback inhibition" may refer to increasing or enhancing the activity of a polypeptide, protein, or enzyme compared to the endogenous activity, or preventing inhibition of activity relative to the endogenous activity. In one specific embodiment, the microorganism may be a microorganism further comprising at least one mutation from mutations that additionally introduce a genetic mutation (R407H) into the *hom* gene (US 10982244 B2), additionally introduce a genetic mutation (T381A, F383A, and/or V323A) into the *ilvA* gene (US 2023-0098971 A1, US 10982244 B2), and additionally introducing a genetic mutation (L377K) into the *lysC* gene encoding aspartate kinase (US 10662450 B2), but is not limited thereto.

In one embodiment, the microorganism having an increased L-amino acid production of the present disclosure may have an increased L-amino acid-producing ability by at least about 1%, at least about 10%, at least about 20%, at least about 30%, at least about 40%, at least about 42.5%, at least about 42.75%, at least about 45%, or at least about 47.5% (the upper limit is not particularly limited and may be, for example, about 200% or less, about 150% or less, about 100% or less, or about 50% or less) as compared to that of the parent strain before modification or a non-modified microorganism, but is not limited thereto. In another embodiment, the microorganism having an increased L-amino acid production of the present disclosure may have an increased L-amino acid-producing ability by at least about 1.01 times, at least about 1.1 times, at least about 1.2 times, at least about 1.3 times, at least about 1.4 times, at least about 1.425 times, at least about 1.4275 times, at least about 1.45 times, or at least about 1.475 times, (the upper limit is not particularly limited and may be, for example, about 10 times or less, about 5 times or less, about 3 times or less, or about 2 times or less) as compared to that of the parent strain before modification or a non-modified microorganism, but is not limited thereto.

As used herein, the term "about" refers to a range which includes all of ±0.5, ±0.4, ±0.3, ±0.2, ±0.1, *etc.,* and includes all of the values that are equivalent or similar in range to the value that follows the term, but the range is not limited thereto.

In one embodiment, the microorganism of the present disclosure may be *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris,* or *Corynebacterium flavescens,* and more specifically, it may be *Corynebacterium glutamicum.*

As used herein, the term "weakening" of a polypeptide is a concept including both having reduced activity or having no activity compared to the endogenous activity. The weakening may be used interchangeably with terms such as inactivation, deficiency, down-regulation, decrease, reduction, and attenuation.

The weakening may also include a case where the activity of the polypeptide itself is reduced or removed compared to the activity of the polypeptide originally possessed by the microorganism due to mutation of a polynucleotide encoding the polypeptide, *etc.;* a case where the overall intracellular polypeptide activity level and/or concentration (expression level) is lower compared to a natural strain due to the inhibition of expression of the gene of a polynucleotide encoding the polypeptide, or the inhibition of translation into the polypeptide, *etc.;* a case where the polynucleotide is not expressed at all; and/or a case where no polypeptide activity is observed even when the polynucleotide is expressed. The term "endogenous activity" refers to the activity of a particular polypeptide originally possessed by a parent strain before transformation or a wild-type or non-modified microorganism, when a trait is altered through genetic modification caused by natural or artificial factors, and may be used interchangeably with "activity before modification". The expression that the polypeptide activity is "inactivated", "deficient", "decreased", "down-regulated", "reduced", or "attenuated" compared to the endogenous activity means that the polypeptide activity is decreased compared to the activity of a particular polypeptide originally possessed by a parent strain before transformation or a non-modified microorganism.

Such weakening of polypeptide activity can be performed by any method known in the art, but the method is not limited thereto, and can be achieved by applying various methods well known in the art (e.g., Nakashima N et al., Bacterial cellular engineering by genome editing and gene silencing. Int J Mol Sci. 2014;15(2):2773-2793, Sambrook et al. Molecular Cloning 2012, *etc*.).

Specifically, the weakening of polypeptide of the present disclosure may result from:
1) deleting a part or all of a gene encoding a polypeptide;
2) modifying an expression regulatory region (or expression regulatory sequence) such that expression of a gene encoding a polypeptide is decreased;
3) modifying an amino acid sequence (e.g., deletion/substitution/addition of at least one amino acid in the amino acid sequence) constituting a polypeptide such that the polypeptide activity is removed or weakened;
4) modifying a gene sequence encoding a polypeptide such that the polypeptide activity is removed or weakened (e.g., deletion/substitution/addition of at least one nucleobase in a nucleobase sequence of a polypeptide gene to encode a polypeptide that has been modified to remove or weaken the polypeptide activity);
5) modifying a nucleotide sequence encoding the initiation codon or 5'-UTR of a gene transcript encoding a polypeptide;
6) introducing an antisense oligonucleotide (e.g., antisense RNA) that complementarily binds to the gene transcript encoding a polypeptide;
7) adding a sequence complementary to a Shine-Dalgarno (SD) sequence of a gene encoding a polypeptide in the upstream of the SD sequence to form a secondary structure incapable of ribosomal attachment;
8) reverse transcription engineering (RTE), which adds a reversely transcribed promoter to the 3' end of an open reading frame (ORF) of a gene sequence encoding a polypeptide;
9) adjusting cellular localization of a protein (polypeptide); or
10) a combination of at least two selected from 1) to 9) above, but is not particularly limited thereto.

For example,
The 1) deleting a part or all of the gene encoding a polypeptide may be deleting all of the polynucleotide encoding an endogenous target polypeptide within the chromosome, replacing with a polynucleotide in which some nucleotides are deleted, or replacing with a marker gene.

In addition, the 2) modifying an expression regulatory region (or expression regulatory sequence) may be inducing a modification on the expression regulatory region (or expression regulatory sequence) through deletion, insertion, non-conservative substitution or conservative substitution, or a combination thereof; or replacing the sequence with a sequence having a weaker activity. The expression regulatory region comprises a promoter, an operator sequence, a sequence encoding a ribosome binding site, and a sequence for regulating the termination of transcription and translation, but is not limited thereto.

In addition, the 3) modifying a nucleotide sequence encoding an initiation codon or 5'-UTR of a gene transcript encoding a polypeptide may be, for example, substituting with a nucleotide sequence encoding another initiation codon having a lower polypeptide expression rate than the endogenous initiation codon, but is not limited thereto.

In addition, the 4) and 5) modifying an amino acid sequence or a polynucleotide sequence may be inducing a modification on the amino acid sequence of the polypeptide or the polynucleotide sequence encoding the polypeptide through deletion, insertion, non-conservative or conservative substitution, or a combination thereof in the sequence to weaken the activity of the polypeptide; or replacing with an amino acid sequence or a polynucleotide sequence modified to have a weaker activity, or an amino acid sequence or a polynucleotide sequence modified to have no activity, but are not limited thereto. For example, the expression of a gene may be inhibited or weakened by introducing a mutation into the polynucleotide sequence to form a termination codon, but is not limited thereto.

The 6) introducing an antisense oligonucleotide (e.g., antisense RNA) that complementarily binds to the gene transcript encoding a polypeptide, can be found in the literature [Weintraub, H. et al., Antisense-RNA as a molecular tool for genetic analysis, Reviews - Trends in Genetics, Vol. 1(1) 1986].

The 7) adding a sequence complementary to a Shine-Dalgarno (SD) sequence of a gene encoding a polypeptide at the front end of the SD sequence to form a secondary structure incapable of ribosomal attachment may be inhibiting mRNA translation or reducing the speed thereof.

The 8) reverse transcription engineering (RTE), which adds a reversely transcribed promoter to the 3' end of an open reading frame (ORF) of a gene sequence encoding a polypeptide, may be forming an antisense nucleotide complementary to the gene transcript encoding a polypeptide to weaken the activity thereof.

The 9) adjusting cellular localization of a protein (polypeptide) may be targeting the protein (polypeptide) to a specific intracellular organelle or to a specific intracellular space. For example, it may be targeting the protein (polypeptide) to the periplasm or the cytoplasm by addition or removal of a leader sequence functioning in protein (polypeptide) targeting, but is not limited thereto.

Such weakening of polypeptide activity may be weakening the activity or concentration expression level of the corresponding polypeptide relative to the activity or concentration of the expressed polypeptide in a wild-type microbial strain or microbial strain before modification, or increasing the amount of product produced from the corresponding polypeptide, but is not limited thereto.

As used herein, the term "enhancement" of the activity of a polypeptide means that the activity of the polypeptide is increased compared to the endogenous activity. The enhancement may be used interchangeably with terms such as activation, up-regulation, overexpression, and increase. Herein, the activation, enhancement, up-regulation, overexpression, and increase may include both exhibition of an activity not originally possessed, or exhibition of an improved activity compared to the endogenous activity or activity before modification. The "endogenous activity" refers to the activity of a particular polypeptide originally possessed by a parent strain before transformation or a non-modified microorganism, when a trait is altered through genetic modification caused by natural or artificial factors, and may be used interchangeably with "activity before modification". The "enhancement", "up-regulation", "overexpression", or "increase" in the activity of a polypeptide compared to the endogenous activity means that the activity and/or concentration (expression level) of the polypeptide is enhanced compared to that of a particular polypeptide originally possessed by a parent strain before transformation or a non-modified microorganism.

The enhancement may be achieved by introducing a foreign polypeptide, or by enhancing the activity and/or concentration (expression level) of the endogenous polypeptide. The enhancement of the activity of a polypeptide can be confirmed by the increase in the level of activity or expression level of the polypeptide, or the amount of product excreted from the polypeptide.

The enhancement of the activity of a polypeptide can employ various methods well known in the art, and the method is not limited as long as it can enhance the activity of the target polypeptide compared to that of a microorganism before modification. Specifically, genetic engineering and/or protein engineering well known to those skilled in the art as common methods in molecular biology may be used, but the method is not limited thereto (e.g., Sitnicka et al. Functional Analysis of Genes. Advances in Cell Biology. 2010, Vol. 2. 1-16, Sambrook et al. Molecular Cloning 2012, *etc*.).

Specifically, the enhancement of polypeptide of the present disclosure may result from:
1) increasing the intracellular copy number of a polynucleotide encoding a polypeptide;
2) replacing an expression regulatory region of a gene encoding a polypeptide on the chromosome with a sequence having a stronger activity;
3) modifying a base sequence encoding the initiation codon or 5'-UTR of a gene transcript encoding a polypeptide;
4) modifying the amino acid sequence of a polypeptide such that the activity of the polypeptide is enhanced;
5) modifying a polynucleotide sequence encoding a polypeptide such that the activity of the polypeptide is enhanced (e.g., modifying a polynucleotide sequence of a polypeptide gene to encode a polypeptide that has been modified to enhance the activity of the polypeptide);
6) introducing a foreign polypeptide exhibiting polypeptide activity or a foreign polynucleotide encoding the same;
7) codon-optimization of a polynucleotide encoding a polypeptide;
8) analyzing the tertiary structure of a polypeptide then selecting and modifying an exposed site, or chemically modifying the same; or
9) adjusting cellular localization of a protein (polypeptide); or
10) a combination of at least two selected from 1) to 9) above, but is not particularly limited thereto.

The 1) increasing the intracellular copy number of a polynucleotide encoding a polypeptide may be achieved by introducing into a host cell a vector operably linked to the polynucleotide encoding the polypeptide and is able to replicate and function regardless of the host. Alternatively, it may be achieved by introducing one copy or at least two copies of the polynucleotide encoding the polypeptide into the chromosome of a host cell. The introduction into the chromosome may be performed by introducing into the host cell a vector capable of inserting the polynucleotide into the chromosome of a host cell, but is not limited thereto. The vector is as described above.

The 2) replacing an expression regulatory region (or expression regulatory sequence) of a gene encoding a polypeptide on the chromosome with a sequence having a strong activity may be, for example, inducing a modification on the sequence through deletion, insertion, non-conservative or conservative substitution, or a combination thereof to further enhance the activity of the expression regulatory region, or replacing the sequence with a sequence having a stronger activity. The expression regulatory region may comprise, but is not particularly limited to, a promoter, an operator sequence, a sequence encoding a ribosome binding site, and a sequence regulating the termination of transcription and translation, *etc.* In one example, it may be replacing the original promoter with a strong promoter, but is not limited thereto.

Examples of known strong promoters include CJ1 to CJ7 promoters (US 7662943 B2), lac promoter, trp promoter, trc promoter, tac promoter, lambda phage PR promoter, PL promoter, tet promoter, gapA promoter, SPL7 promoter, SPL13 (sm3) promoter (US 10584338 B2), O2 promoter (US 10273491 B2), tkt promoter, yccA promoter, *etc.,* but the strong promoter is not limited thereto.

The 3) modifying a base sequence encoding the initiation codon or 5'-UTR of a gene transcript encoding a polypeptide may, for example, substituting with a base sequence encoding another initiation codon having a higher expression rate of the polypeptide compared to the endogenous initiation codon, but is not limited thereto.

The 4) and 5) modifying an amino acid sequence or a polynucleotide sequence may be inducing a modification on the amino acid sequence of the polypeptide or the polynucleotide sequence encoding the polypeptide through deletion, insertion, non-conservative or conservative substitution, or a combination thereof in the sequence to enhance the activity of the polypeptide; or replacing with an amino acid sequence or a polynucleotide sequence modified to have a stronger activity, or an amino acid sequence or a polynucleotide sequence modified to have enhanced activity, but are not limited thereto. The replacement may specifically be performed by inserting the polynucleotide into the chromosome by homologous recombination, but is not limited thereto. The vector used herein may further comprise a selection marker for confirming the insertion into the chromosome. The selection marker is as described above.

The 6) introducing a foreign polynucleotide exhibiting polypeptide activity may be introducing into a host cell a foreign polynucleotide encoding a polypeptide that exhibits the same or similar activity to that of the polypeptide. The foreign polynucleotide may be used without limitation to the origin or sequence, as long as it exhibits the same or similar activity to that of the polypeptide. The introduction may be performed by those skilled in the art by appropriately selecting a transformation method known in the art, and when the introduced polynucleotide is expressed in a host cell, a polypeptide is produced and the activity thereof may be increased.

The 7) codon-optimization of a polynucleotide encoding a polypeptide may be codon-optimization of an endogenous polynucleotide to increase the transcription or translation within a host cell, or codon-optimization of the foreign polynucleotide such that optimal transcription and translation can be achieved within the host cell.

The 8) analyzing the tertiary structure of a polypeptide then selecting and modifying an exposed site, or chemically modifying the same may be, for example, comparing the sequence information of a polypeptide to be analyzed with a database storing sequence information of known proteins to determine template protein candidates according to the degree of sequence similarity, and thus confirming the structure based on the information to select and transform or modify the exposed site to be modified or chemically modified.

The 9) adjusting cellular localization of a protein (polypeptide) may be targeting a protein (polypeptide) to a specific intracellular organelle or to a specific intracellular space. For example, it may be targeting a protein (polypeptide) to the periplasm or the cytoplasm by addition or removal of a leader sequence functioning in protein (polypeptide) targeting, but is not limited thereto.

Such enhancement of polypeptide activity may be an increase in the activity or concentration expression level of the corresponding polypeptide relative to the activity or concentration of the polypeptide expressed in a wild-type microbial strain or a microbial strain before modification, or an increase in the amount of product produced from the polypeptide, but is not limited thereto.

The modification of a part or all of a polynucleotide in the microorganism of the present disclosure (e.g., modification for encoding the protein variant described above) may be induced by (a) homologous recombination using a vector for chromosomal insertion in the microorganism or genome editing using an engineered nuclease (*e.g.,* CRISPR-Cas9), and/or (b) light, such as ultraviolet rays and radiation, *etc.* and/or chemical treatments, but is not limited thereto. The method of modifying a part or all of the gene may include a method using DNA recombination technology. For example, a part or all of the gene may be deleted by injecting a nucleotide sequence or a vector comprising a nucleotide sequence homologous to a target gene into the microorganism to induce homologous recombination. The injected nucleotide sequence or vector may comprise a dominant selection marker, but is not limited thereto.

In the microorganism of the present disclosure, the protein variant, polynucleotide, L-amino acid, *etc.* are as described in other aspects above.

Still another aspect of the present disclosure provides a method for producing an L-amino acid, comprising culturing a microorganism of the genus *Corynebacterium,* comprising the protein variant of the present disclosure or the polynucleotide of the present disclosure, in a medium.

The method for producing an L-amino acid of the present disclosure may comprise culturing a microorganism of the genus *Corynebacterium,* comprising the protein variant of the present disclosure, the polynucleotide of the present disclosure, or the vector of the present disclosure, in a medium.

As used herein, the term "culturing" means growing the microorganism of the genus *Corynebacterium* of the present disclosure under appropriately controlled environmental conditions. The culturing process of the present disclosure may be performed according to a suitable medium and culturing conditions known in the art. Such a culturing process may be easily adjusted for use by those skilled in the art according to the selected strain. Specifically, the culturing may be a batch type, continuous type, and/or fed-batch type, but is not limited thereto.

As used herein, the term "medium" refers to a material in which nutrients required for culturing the microorganism of the genus *Corynebacterium* of the present disclosure are mixed as main components, and supplies nutrients, growth factors, *etc.* including water, which is essential for survival and growth. Specifically, the medium and other culturing conditions used for culturing the microorganism of the genus *Corynebacterium* of the present disclosure may be any medium used for conventional culturing of microorganisms without any particular limitation, but the microorganism of the genus *Corynebacterium* of the present disclosure may be cultured under aerobic conditions in a common medium containing an appropriate carbon source, nitrogen source, phosphorus source, inorganic compound, amino acid, and/or vitamin, by adjusting the temperature, pH, *etc.*

Specifically, the culture medium for the microorganism of the genus *Corynebacterium* can be found in the literature ["Manual of Methods for General Bacteriology" by the American Society for Bacteriology (Washington D.C., USA, 1981)].

In the present disclosure, the carbon source may include carbohydrates, such as glucose, saccharose, lactose, fructose, sucrose, maltose, *etc.;* sugar alcohols, such as mannitol, sorbitol, *etc.;* organic acids, such as pyruvic acid, lactic acid, citric acid, *etc.;* amino acids, such as glutamic acid, methionine, lysine, *etc.;* or the like. Additionally, natural organic nutrients such as starch hydrolysate, molasses, blackstrap molasses, rice bran, cassava, sugar cane residues, and corn steep liquor may be used, and specifically, carbohydrates such as glucose and sterilized pretreated molasses (*i.e.,* molasses converted to reducing sugar), *etc.* may be used. Additionally, various other carbon sources in an appropriate amount may be used without limitation. These carbon sources may be used alone or in a combination of at least two kinds, but are not limited thereto.

The nitrogen source may include inorganic nitrogen sources, such as ammonia, ammonium sulfate, ammonium chloride, ammonium acetate, ammonium phosphate, ammonium carbonate, ammonium nitrate, *etc.;* and organic nitrogen sources, such as amino acids, such as glutamic acid, methionine, glutamine, *etc.,* peptone, NZ-amine, meat extract, yeast extract, malt extract, corn steep liquor, casein hydrolysate, fish or decomposition product thereof, defatted soybean cake or decomposition product thereof, *etc.* These nitrogen sources may be used alone or in a combination of at least two kinds, but are not limited thereto.

The phosphorus source may include monopotassium phosphate, dipotassium phosphate, or sodium-containing salts corresponding thereto. As for the inorganic compound, sodium chloride, calcium chloride, iron chloride, magnesium sulfate, iron sulfate, manganese sulfate, calcium carbonate, *etc*. may be used. Additionally, amino acids, vitamins, and/or appropriate precursors, *etc.* may be included. These components or precursors may be added to a medium in a batch or continuous manner. However, the medium is not limited thereto.

Additionally, the pH of the medium may be adjusted by adding a compound such as ammonium hydroxide, potassium hydroxide, ammonia, phosphoric acid, sulfuric acid, *etc.* to the medium in an appropriate manner during the culturing of the microorganism of the genus *Corynebacterium* of the present disclosure. Further, during the culturing, foaming may be suppressed by using an antifoaming agent such as fatty acid polyglycol ester. Additionally, oxygen or oxygen-containing gas may be injected into the medium in order to maintain the aerobic state of the medium, or gas may not be injected or nitrogen, hydrogen, or carbon dioxide gas may be injected in order to maintain the anaerobic and microaerobic states, but the medium is limited thereto.

The culture temperature during the culturing of the present disclosure may be in the range from 20°C to 45°C, specifically 25°C to 40°C, 25°C to 40°C, 25°C to 37°C, 25°C to 35°C, 27°C to 40°C, 27°C to 37°C, 27°C to 35°C, 30°C to 40°C, 30°C to 37°C, or 30°C to 35°C, but is not limited thereto. In the culturing of the present disclosure, the culturing period may be about 10 to 160 hours, but is not limited thereto.

The L-amino acid produced by the culturing of the present disclosure may be secreted into the medium or remain in the cells.

The method for producing an L-amino acid of the present disclosure may further comprise preparing the microorganism of the genus *Corynebacterium* of the present disclosure, preparing a medium for culturing the strain, or a combination thereof (in any order), for example, prior to the culturing.

The method for producing an L-amino acid of the present disclosure may further comprise recovering L-amino acid from the culture medium (medium in which the culture was grown) or the microorganism of the genus *Corynebacterium.* The recovery may be further comprised after the culturing.

The recovery may be collecting the target L-amino acid using a suitable method known in the art according to the culturing method of the microorganism of the present disclosure, for example, a batch, continuous, or fed-batch type culturing method, *etc.* For example, centrifugation, filtration, treatment with a crystallized protein precipitating agent (salting-out), extraction, sonication, ultrafiltration, dialysis, various types of chromatography such as molecular sieve chromatography (gel filtration), adsorption chromatography, ion exchange chromatography, affinity chromatography, *etc.,* HPLC, and a combination of these methods may be used, and the target L-amino acid can be recovered from the medium or the microorganism using suitable methods known in the art.

Further, the method for producing an L-amino acid of the present disclosure may further comprise purification. The purification may be performed using an appropriate method known in the art. In one example, when the method for producing an L-amino acid of the present disclosure comprises both recovery and purification, the recovery and purification may be performed continuously or non-continuously regardless of the order, or integrated into a simultaneous or a single step, but the method is not limited thereto.

In the method of the present disclosure, the variant, polynucleotide, vector, microorganism, *etc.,* are as described in other aspects above.

Still another aspect of the present disclosure provides a composition for producing an L-amino acid, comprising: a microorganism of the genus *Corynebacterium* comprising at least one selected from the group consisting of the protein variant of the present disclosure, a polynucleotide encoding the protein variant, or a vector comprising the polynucleotide; a medium in which the microorganism has been cultured; or a combination of at least two thereof.

The composition of the present disclosure may further comprise any suitable excipient commonly used in compositions for producing amino acids, and such excipients may be, for example, preservatives, wetting agents, dispersing agents, suspending agents, buffers, stabilizers, isotonic agents, *etc.,* but are not limited thereto.

In the composition of the present disclosure, the variant, polynucleotide, vector, strain, medium, L-amino acid, *etc.,* are as described in other aspects above.

According to another aspect of the present disclosure, the present disclosure may provide a method for increasing L-amino acid-producing ability of the microorganism, a method for imparting L-amino acid-producing ability to the microorganism, or a method for preparing a microorganism having an increased L-amino acid-producing ability, wherein the method comprises introducing (e.g., transformation) into a microorganism the novel protein variant of the present disclosure, a polynucleotide encoding the variant, and/or a recombinant vector comprising the polynucleotide.

In the method for preparing a microorganism of the present disclosure, the polynucleotide, recombinant vector, microorganism, *etc.,* are as described above.

Still another aspect of the present disclosure provides a use of the protein variant of the present disclosure, a polynucleotide encoding the protein variant, or a microorganism comprising at least one selected from the group consisting of the protein variant and a polynucleotide encoding the protein variant for producing an L-amino acid.

In the use of the present disclosure, the protein variant, polynucleotide, microorganism, *etc.,* are as described above.

### [Mode for Carrying Out the Invention]

Hereinafter, the present disclosure will be described in more detail by way of Examples. However, the following Examples are merely preferred embodiments given for illustrative purposes, and thus, the scope of the present disclosure is not intended to be limited to or by these Examples. Meanwhile, technical matters not described herein may be sufficiently understood and easily implemented by those skilled in the technical field of the present disclosure or similar technical fields.

### Examples

### Example 1. Construction of Threonine Biosynthetic Gene Cluster (thrABC) Variant Library

### Example 1-1. Construction of pDC24ΔNCgl1496 Vector for Insertion of Target Gene

To insert a foreign gene into the chromosome of *Corynebacterium glutamicum,* NCgl1496, which is known as a gene encoding a transposon in *Corynebacterium glutamicum,* was used as an insertion site.

Specifically, in order to construct an NCgl1496 deletion and target gene insertion vector, PCR was performed using genomic DNA of the wild-type *Corynebacterium glutamicum* ATCC13032 strain as a template and primer pairs of SEQ ID NOs: 1 and 2, and SEQ ID NOs: 3 and 4, thereby obtaining a left homology arm region of NCgl1496 and a right homology arm region of NCgl1496, respectively. Solg^{™} Pfu-X DNA polymerase (SolGent co.) was used as the polymerase for the PCR reaction, and the PCR was performed under conditions of denaturation at 95°C for 5 minutes, followed by 27 cycles of denaturation at 95°C for 20 seconds, annealing at 55°C for 40 seconds, and polymerization at 72°C for 1 minute, and then polymerization at 72°C for 5 minutes. The primers of SEQ ID NOs: 2 and 3 were designed to comprise a Scal-HF restriction site between the left homology arm and the right homology arm. The primer sequences used herein are shown in Table 2 below.

**[Table 2]**

| SEQ ID NO. | Sequence Name | Sequence (5'->3') |
|---|---|---|
| 1 | N1496 Left F | TCGAGCTCGGTACCCGTTCCATCGCTGAGT |
| 2 | N1496 Left R | TGGTTTTTCGAAAGTACTACCTGATTATTCCA |
| 3 | N1496 Right F | TGGAATAATCAGGTAGTACTTTCGAAAAACCA |
| 4 | N1496 Right R | AGAGGATCCCCTGAGGAGTACATGCGCGAC |

The gene fragments obtained by the above process were cloned into a pDC24 vector (SEQ ID NO: 5) digested with the Smal restriction enzyme using the Gibson Assembly method (DG Gibson et al., Nature Methods, Vol. 6, No. 5, May 2009; NEBuilder HiFi DNA Assembly Master Mix), thereby obtaining a recombinant plasmid. The cloning was performed by mixing the Gibson Assembly reagent and each gene fragment at calculated molar amounts and incubating the mixture at 50°C for 1 hour. Through this process, an NCgl1496 gene deletion and target gene insertion vector was constructed, and was named pDC24ΔNCgl1496.

### Example 1-2. Construction of Threonine Biosynthetic Gene Cluster (thrABC) Variant Expression Vector Library by Artificial Mutagenesis

The *thrABC* variant expression vector library was constructed by the following method.

Using the genomic DNA of *Escherichia coli W* ATCC9637 (NC_017635) strain as a template, *thrABC* gene fragments into which base substitution mutations were randomly introduced were obtained by an error-prone PCR method using a primer pair of SEQ ID NOs: 6 and 7. Error-prone PCR was performed using a GeneMorphll Random Mutagenesis Kit (Stratagene) under conditions in which 0 to 3.5 mutations per kb were introduced into the amplified gene fragments, and the PCR was performed under conditions of 30 cycles of denaturation at 96°C for 30 seconds, annealing at 53°C for 30 seconds, and polymerization at 72°C for 6 minutes. In order to obtain a gapA promoter (PgapA) fragment linkable to *thrABC,* PCR was performed using the genomic DNA of the wild-type *Corynebacterium glutamicum* ATCC13032 strain as a template and a primer pair of SEQ ID NOs: 8 and 9, thereby obtaining the PgapA fragment. The PCR was performed under conditions of denaturation at 95°C for 5 minutes, followed by 27 cycles of denaturation at 95°C for 20 seconds, annealing at 55°C for 40 seconds, and polymerization at 72°C for 30 seconds, and then polymerization at 72°C for 5 minutes. The primer sequences used herein are shown in Table 3 below.

**[Table 3]**

| SEQ ID NO. | Sequence Name | Sequence (5'->3') |
|---|---|---|
| 6 | thrABC F | AGAGGAGACACAACATGCGAGTGTTGAAGT |
| 7 | thrABC R | GTGGTTTTTCGAAAGTTTACTGATGATTCA |
| 8 | PgapA F | GGAATAATCAGGTAGTTTAATTTGTGGATT |
| 9 | PgapA R | ACTTCAACACTCGCATGTTGTGTCTCCTCT |

After the pDC24ΔNCgl1496 vector constructed in Example 1-1 was treated with the Scal-HF restriction enzyme, the variant *thrABC* fragments and PgapA fragment obtained through the above process were cloned using a Gibson Assembly method in the same manner as in Example 1-1, and was then transformed into *Escherichia coli* DH5α and spread onto an LB solid medium comprising kanamycin (25 mg/L).

After selecting 20 transformed colonies, plasmids were obtained and subjected to nucleotide sequence analysis, which confirmed that mutations were introduced at different positions at an average frequency of 1.5 mutations per kb. Plasmids were extracted from the transformed *Escherichia coli* colonies, and the resulting collection was named pDC24ΔNCgl1496-PgapA_thrABC(mt) library.

### Example 1-3. Construction of Threonine Biosynthetic Gene Cluster (thrABC) Wild-type Vector

In order to construct pDC24ΔNCgl1496-PgapA_thrABC(WT) vector as a control, PCR was performed in the same manner as in Example 1-1 using genomic DNA of *Escherichia coli W* ATCC9637 (NC_017635) as a template and a primer pair of SEQ ID NOs: 6 and 7, thereby obtaining a wild-type *thrABC* gene fragment. Subsequently, in order to obtain a gapA promoter (PgapA) fragment linkable to the *thrABC* fragment, PCR was performed in the same manner as in Example 1-1 using genomic DNA of the wild-type *Corynebacterium glutamicum* ATCC13032 strain as a template and a primer pair of SEQ ID NOs: 8 and 9, thereby obtaining the PgapA fragment.

After the pDC24ΔNCgl1496 vector constructed in Example 1-1 was treated with the Scal-HF restriction enzyme, the wild-type *thrABC* fragments and PgapA fragment obtained through the above process were cloned using a Gibson Assembly method in the same manner as in Example 1-1, thereby obtaining the recombinant plasmid. The constructed recombinant plasmid was named pDC24ΔNCgl1496-PgapA_thrABC(WT).

### Example 2. Selection of Threonine Biosynthetic Gene Cluster (thrABC) Variants through Library Screening

### Example 2-1. Construction of a Variant Strain Library with Increased L-Threonine-Producing Ability

Using a threonine-producing strain KCCM12502P (Korean Patent No. 10-2126951) as the parent strain, the pDC24ΔNCgl1496-PgapA_*thrABC*(mt) library constructed above was transformed by homologous chromosomal recombination via electroporation (Appl. Microbiol. Biotechnol. (1999) 52: 541-545), and spread onto a complex plate medium comprising kanamycin (25 mg/L) to obtain approximately 10,000 colonies. These colonies were named KCCM12502PΔNCgl1496:: PgapA_thrABC(mt)-1 to KCCM12502PΔNCgl1496:: PgapA_thrABC(mt)-10000. In addition, the constructed pDC24ΔNCgl1496-PgapA_thrABC(WT) vector was transformed into the KCCM12502P strain to prepare a control strain, which was named KCCM12502PΔNCgl1496:: PgapA_thrABC(WT).

### <Complex Plate Medium (pH 7.0)>

Glucose 10 g, Peptone 10 g, Beef extract 5 g, Yeast extract 5 g, Brain Heart Infusion 18.5 g, NaCl 2.5 g, Urea 2 g, Sorbitol 91 g, Agar 20 g (based on 1 L of distilled water)

### Example 2-2. Screening of a Variant Strain Library with Increased L-Threonine-Producing Ability

Approximately 10,000 colonies obtained in Example 2-1 and the control strain were each inoculated into 300 µL of a selection medium comprising the components described below, and cultured in a 96-deep well plate at 32°C and 1000 rpm for about 24 hours. In order to analyze the amount of L-threonine produced during culture, ninhydrin method was used (J. Biol. Chem. 1948, 176: 367-388). After completion of culture, 10 µL of the culture supernatant was reacted with 190 µL of a ninhydrin reaction solution (63% glycerol, 27% ninhydrin solution (7.1 g/L in 0.5 M citrate buffer, pH 5.5)) at 65°C for 30 minutes, and then the absorbance was measured at a wavelength of 570 nm using a spectrophotometer. Approximately 300 variant strain colonies exhibiting an absorbance increased by at least 10% compared to that of the control strain KCCM12502PΔNCgl1496:: PgapA_thrABC(WT) were selected. The remaining colonies exhibited absorbance values similar to or lower than that of the control. The composition of the selection medium is as follows.

### <Selection Medium (pH 8.0)>

Glucose 10 g, Ammonium sulfate 5.5 g, MgSO₄·7H₂O 1.2 g, KH₂PO₄ 0.8 g, K₂HPO₄ 16.4 g, Biotin 100 µg, Thiamine HCl 1000 µg, Calcium Pantothenate 2000 µg, Nicotinamide 2000 µg (based on 1 L of distilled water)

The above method was repeatedly performed on the 300 strains selected above, and the top 30 variant strains exhibiting improved L-threonine-producing ability compared to the KCCM12502PΔNCgl1496:: PgapA_thrABC(WT) strain were selected.

### Example 2-3. Evaluation of L-Threonine-Producing Ability of Selected Variant Strains

The 30 variant strains selected in Example 2-2 were cultured, and the L-threonine-producing abilities thereof were analyzed as follows.

Each strain was inoculated into a 250 mL corner-baffle flask containing 25 mL of a seed medium and shaking cultured at 30°C and 200 rpm for 20 hours. Then, 1 mL of the seed culture was inoculated into a 250 mL corner-baffle flask containing 24 mL of a production medium and shaking cultured at 32°C and 200 rpm for 72 hours. The concentration of L-threonine was analyzed using high-performance liquid chromatography (HPLC).

### <Seed Medium (pH 7.0)>

Glucose 20 g, Peptone 10 g, Yeast extract 5 g, Urea 1.5 g, KH₂PO₄ 4 g, K₂HPO₄ 8 g, MgSO₄·7H₂O 0.5 g, Biotin 0.1 mg, Thiamine HCl 1 mg, Calcium pantothenate 22 mg, Nicotinamide 2 mg (based on 1 L of distilled water)

### <Production Medium (pH 7.0)>

Glucose 63 g, (NH₄)₂SO₄ 28 g, Soy protein 20 g, Molasses 14 g, KH₂PO₄ 1.1 g, MgSO₄·7H₂O 1.2 g, Biotin 1.8 mg, Thiamine HCl 9 mg, Calcium Pantothenate 9 mg, MnSO₄ 180 mg, FeSO₄ 200 mg, ZnSO₄ 1 mg, CuSO₄ 1 mg, CaCO₃ 30 g (based on 1 L of distilled water)

Among the 30 variant strains selected above, the top 6 strains exhibiting increased L-threonine concentration compared to the control were selected. The analyzed L-threonine concentrations are as shown in Table 4 below.

**[Table 4]**

| Strain Name | L-Threonine (g/L) |
|---|---|
| KCCM12502P | 3.2 |
| KCCM12502PΔNCgl1496::PgapA_thrABC(WT) | 4.0 |
| KCCM12502PΔNCgl1496::PgapA_thrABC(mt)-1 | 4.5 |
| KCCM12502PΔNCgl1496::PgapA_thrABC(mt)-2 | 4.5 |
| KCCM12502PΔNCgl1496::PgapA_thrABC(mt)-3 | 4.3 |
| KCCM12502PΔNCgl1496::PgapA_thrABC(mt)-4 | 4.3 |
| KCCM12502PΔNCg11496::PgapA_thrABC(mt)-5 | 4.2 |
| KCCM12502PΔNCg11496::PgapA_thrABC(mt)-6 | 4.1 |

As shown in the table above, when a wild-type *thrABC* gene derived from *Escherichia coli* was introduced, it was confirmed that threonine production increased compared to the parent strain KCCM12502P. In addition, when a variant *thrABC* gene was introduced, threonine production was increased by about 102.5% to about 112% compared to KCCM12502P ΔNCgl1496:: *PgapA_thrABC*(WT) into which the wild-type *thrABC* gene had been introduced. From these results, it was found that *thrABC* mutation improves the activity of ThrABC, thereby enhancing the L-threonine-producing ability of the strain.

### Example 2-4. Confirmation of Threonine Biosynthetic Gene Cluster (thrABC) Mutation in Selected Variant Strains

In order to identify mutations introduced into *thrABC* in the strains selected in Example 2-3, nucleotide sequence analysis was performed. PCR was individually performed using each genomic DNA of six strains selected for sequence determination as templates and a primer pair of SEQ ID NOs: 10 and 11. The PCR was performed under conditions of denaturation at 95°C for 5 minutes, followed by 27 cycles of denaturation at 95°C for 20 seconds, annealing at 60°C for 40 seconds, and polymerization at 72°C for 6 minutes, and then polymerization at 72°C for 5 minutes. The primer sequences used herein are shown in Table 5 below.

**[Table 5]**

| SEQ ID NO. | Sequence Name | Sequence (5'->3') |
|---|---|---|
| 10 | thrABC SEQ F | GCTGCGAAATCTTTGTTTCC |
| 11 | thrABC SEQ R | CCCAAACACCTCCCGTTTAT |

Through nucleotide sequence analysis of the variant *thrABC* of each of the six selected strains, the amino acid sequences of variant ThrA, ThrB, and ThrC were identified and compared with the amino acid sequences of wild-type ThrA, ThrB, and ThrC (SEQ ID NOs: 12, 13, and 14). Variant in which changes in protein sequences occurred due to nucleotide sequence mutations within the variant *thrABC* of the six selected strains are disclosed in Table 6 below.

**[Table 6]**

| Strain Name | Variant Name | SEQ ID NO. |
|---|---|---|
| KCCM12502PΔNCgl1496:: PgapA_thrABC(mt)-1 | ThrA(S352P,G433R,W521R) | 15 |
| KCCM12502PΔNCgl1496:: PgapA_thrABC(mt)-2 | ThrA(Q58R,S352P,G433R,W521R,N710D) | 16 |
| KCCM12502PΔNCgl1496:: PgapA_thrABC(mt)-3 | ThrA(S352P,G433R,W521R,N710D) | 17 |
| KCCM12502PΔNCgl1496:: PgapA_thrABC(mt)-4 | ThrA(Q58R,S352P,G433R,W521R,N710D, Y784C) | 18 |
| KCCM12502PΔNCgl1496:: PgapA_thrABC(mt)-5 | ThrB(Q247R) | 19 |
| KCCM12502PΔNCgl1496:: | ThrC(V193A) | 20 |
| PgapA_thrABC(mt)-6 | | |

As a result, it was found that as few as one amino acid and as many as six amino acids were substituted.

### Example 3. Introduction of Threonine Biosynthetic Gene Cluster (thrABC) Mutation in an Isoleucine-Producing Strain and Evaluation thereof

### Example 3-1. Construction of Recombinant Vector for Introduction of Threonine Biosynthetic Gene Cluster (thrABC) Mutation

In order to confirm whether introduction of the ThrABC variants selected in Example 2 into a *Corynebacterium glutamicum* strain having L-isoleucine-producing ability results in an increased isoleucine-producing ability, a vector for variant introduction was constructed by the following method.

Specifically, PCR was performed in the same manner as in Example 2-4 using genomic DNAs of KCCM12502PΔNCgl1496:: PgapA_thrABC(mt)-1, KCCM12502PΔNCgl1496:: PgapA_thrABC(mt)-2, KCCM12502PΔNCgl1496:: PgapA_thrABC(mt)-3, KCCM12502PΔNCgl1496:: PgapA_thrABC(mt)-4, KCCM12502PΔNCgl1496:: PgapA_thrABC(mt)-5, and KCCM12502PΔNCgl1496:: PgapA_thrABC(mt)-6 selected in Example 2-4 as templates and a primer pair of SEQ ID NOs: 7 and 8, thereby obtaining PgapA_thrA(S352P, G433R, W521R)BC, PgapA_thrA(Q58R, S352P, G433R, W521R, N710D)BC, PgapA_thrA(S352P, G433R, W521R, N7100)BC, PgapA_thrA(Q58R, S352P, G433R, W521R, N710D, Y784C)BC, PgapA_thrAB(Q247R)C, and PgapA_thrABC(V193A) gene fragments.

After the pDC24ΔNCgl1496 vector constructed in Example 1-1 was treated with the Scal-HF restriction enzyme, the PgapA_thrA(S352P, G433R, W521R)BC, PgapA_thrA(Q58R, S352P, G433R, W521R, N710D)BC, PgapA_thrA(S352P, G433R, W521R, N7100)BC, PgapA_thrA(Q58R, S352P, G433R, W521R, N710D, Y784C)BC, PgapA_thrAB(Q247R)C, and PgapA_thrABC(V193A) gene fragments obtained through the above process were cloned using a Gibson Assembly method in the same manner as in Example 1-1, thereby obtaining each respective recombinant plasmid. The constructed recombinant plasmids were named pDC24ΔNCgl1496-PgapA_thrA(S352P, G433R, W521R)BC, pDC24ΔNCgl1496-PgapA_thrA(Q58R, S352P, G433R, W521R, N710D)BC, pDC24ΔNCgl1496-PgapA_thrA(S352P, G433R, W521R, N710D)BC, pDC24ΔNCgl1496-PgapA_thrA(Q58R, S352P, G433R, W521R, N710D, Y784C)BC, pDC24ΔNCgl1496-PgapA_thrAB(Q247R)C, and pDC24ΔNCgl1496-PgapA_thrABC(V193A).

### Example 3-2. Construction of an Isoleucine-Producing Strain Introduced with Threonine Biosynthetic Gene Cluster (thrABC) Mutation

The recombinant vectors pDC24ΔNCgl1496-PgapA_thrA(S352P, G433R, W521R)BC, pDC24ΔNCgl1496-PgapA_thrA(Q58R, S352P, G433R, W521R, N710D)BC, pDC24ΔNCgl1496-PgapA_thrA(S352P, G433R, W521R, N710D)BC, pDC24ΔNCgl1496-PgapA_thrA(Q58R, S352P, G433R, W521R, N710D, Y784C)BC, pDC24ΔNCgl1496-PgapA_thrAB(Q247R)C, and pDC24ΔNCgl1496-PgapA_thrABC(V193A) constructed in Example 3-2, and the recombinant vector pDC24ΔNCgl1496-PgapA_thrABC(WT) constructed in Example 1 were each transformed into an isoleucine-producing strain KCCM12739P (Korean Patent No. 10-2363913) by electroporation. Transformed strains were obtained from selection media comprising kanamycin (25 mg/L), and were named KCCM12739P ΔNCgl1496 :: PgapA_thrA(S352P, G433R, W521R)BC, KCCM12739P ΔNCgl1496 :: PgapA_thrA(Q58R, S352P, G433R, W521R, N710D)BC, KCCM12739P ΔNCgl1496 :: PgapA_thrA(S352P, G433R, W521R, N710D)BC, KCCM12739P ΔNCgl1496 :: PgapA_thrA(Q58R, S352P, G433R, W521R, N710D, Y784C)BC, KCCM12739P ΔNCgl1496 :: PgapA_thrAB(Q247R)C, KCCM12739P ΔNCgl1496 :: PgapA_thrABC(V193A), and KCCM12739P ΔNCgl1496 :: PgapA_thrABC(WT), respectively.

### Example 3-3. Evaluation of Isoleucine-Producing Ability

In order to confirm their L-isoleucine-producing abilities, the strains prepared in Example 3-2 were cultured and evaluated as follows. The parent strain and the variant strains were inoculated into a 250 mL corner-baffle flask containing 25 mL of an isoleucine production medium, and shaking cultured at 32°C and 200 rpm for 60 hours.

### <Production Medium (pH 7.2)>

Glucose 10%, Yeast extract 0.2%, (NH₄)₂SO₄ 1.6%, KH₂PO₄ 0.1%, MgSO₄·7H₂O 0.1%, FeSO₄·7H₂O 10 mg/L, MnSO₄·H₂O 10 mg/L, Biotin 200 µg/L (based on 1 L of distilled water)

After completion of the culture, the L-isoleucine-producing abilities were measured using HPLC, and the analyzed L-isoleucine concentrations are as shown in Table 7 below.

**[Table 7]**

| Strain Name | L-Isoleucine (g/L) |
|---|---|
| KCCM12739P | 3.0 |
| KCCM12739PΔNCgl1496::PgapA_thrABC(WT) | 3.8 |
| KCCM12739PΔNCgl1496::PgapA_thrA(S352P,G433R,W521R)BC | 4.3 |
| KCCM12739PΔNCgl1496::PgapA_thrA(Q58R,S352P,G433R,W521R,N7 10D)BC | 4.4 |
| KCCM12739PΔNCgl1496::PgapA_thrA(S352P,G433R,W521R,N710D)B C | 4.2 |
| KCCM12739PΔNCgl1496::PgapA_thrA(Q58R,S352P,G433R,W521R,N7 100,Y784C)BC | 4.2 |
| KCCM12739PΔNCgl1496::PgapA_thrAB(Q247R)C | 4.1 |
| KCCM12739PΔNCgl1496::PgapA_thrABC(V193A) | 3.9 |

As shown in the table above, when a wild-type *thrABC* gene derived from *Escherichia coli was* introduced, it was confirmed that isoleucine production increased compared to the parent strain KCCM12739P. In addition, when a variant *thrABC* gene was introduced, isoleucine production was increased by about 102% to about 113% compared to KCCM12739P ΔNCgl1496:: PgapA_thrABC(WT) into which the wild-type *thrABC* gene had been introduced. In particular, isoleucine-producing abilities in KCCM12739PΔNCgl1496:: PgapA_thrA(S352P, G433R, W521R)BC, KCCM12739PΔNCgl1496:: PgapA_thrA(Q58R, S352P, G433R, W521R, N710D)BC, KCCM12739PΔNCgl1496::PgapA_thrA(S352P,G433R,W521 R,N710D)BC, and KCCM12739PΔNCgl1496::PgapA_thrA(Q58R,S352P,G433R,W521R,N710D,Y784C) BC greatly increased compared to the parent strain KCCM12739P. From these results, it was found that *thrABC* mutation improves the activity of ThrABC, thereby enhancing the L-isoleucine-producing ability of the strain.

### Example 4. Introduction of Aspartokinase and Homoserine Dehydrogenase 1 (ThrA) Mutation in an O-Acetyl Homoserine-Producing Strain and Evaluation thereof

### Example 4-1. Construction of Recombinant Vector for Introduction of Aspartokinase and Homoserine Dehydrogenase 1 (ThrA) Mutation

In order to confirm whether introduction of the ThrA variants selected in Example 2 into a *Corynebacterium glutamicum* strain having O-acetyl homoserine-producing ability results in an increased O-acetyl homoserine-producing ability, a vector for variant introduction was constructed by the following method.

Specifically, PCR was performed using genomic DNAs of KCCM12502PΔNCgl1496:: PgapA_thrABC(mt)-1, KCCM12502PΔNCgl1496:: PgapA_thrABC(mt)-2, KCCM12502PΔNCgl1496:: PgapA_thrABC(mt)-3, and KCCM12502PΔNCgl1496:: PgapA_thrABC(mt)-4 selected in Example 2-4 as templates and a primer pair of SEQ ID NOs: 8 and 21, thereby obtaining PgapA_thrA(S352P, G433R, W521R), PgapA_thrA(Q58R, S352P, G433R, W521R, N710D), PgapA_thrA(S352P, G433R, W521R, N710D), and PgapA_thrA(Q58R, S352P, G433R, W521R, N710D, Y784C) gene fragments. In addition, in order to construct the pDC24ΔNCgl1496-PgapA_thrA(WT) vector as a control, PCR was performed using the genomic DNA of the KCCM12502PΔNCgl1496:: PgapA_thrABC(WT) strain constructed in Example 2-1 as a template and a primer pair of SEQ ID NOs: 8 and 21, thereby obtaining the PgapA_thrA(WT) gene fragment. The PCR was performed under conditions of denaturation at 95°C for 5 minutes, followed by 27 cycles of denaturation at 95°C for 5 seconds, annealing at 60°C for 40 seconds, and polymerization at 72°C for 4 minutes, and then polymerization at 72°C for 5 minutes.

After the pDC24ΔNCgl1496 vector constructed in Example 1-1 was treated with the Scal-HF restriction enzyme, the PgapA_thrA(S352P, G433R, W521R), PgapA_thrA(Q58R, S352P, G433R, W521R, N710D), PgapA_thrA(S352P, G433R, W521R, N710D), PgapA_thrA(Q58R, S352P, G433R, W521R, N710D, Y784C), and PgapA_thrA(WT) gene fragments obtained through the above process were cloned using a Gibson Assembly method in the same manner as in Example 1-1, thereby obtaining each respective recombinant plasmid. The constructed recombinant plasmids were named pDC24ΔNCgl1496-PgapA_thrA(S352P, G433R, W521R), pDC24ΔNCgl1496-PgapA_thrA(Q58R, S352P, G433R, W521R, N710D), pDC24ΔNCgl1496-PgapA_thrA(S352P, G433R, W521R, N710D), pDC24ΔNCgl1496-PgapA_thrA(Q58R, S352P, G433R, W521R, N710D, Y784C), and pDC24ΔNCgl1496-PgapA_ThrA(WT).

### Example 4-2. Construction of an O-Acetyl Homoserine-Producing Strain Introduced with Aspartokinase and Homoserine Dehydrogenase 1 (ThrA) Mutation

The recombinant vectors pDC24ΔNCgl1496-PgapA_thrA(S352P, G433R, W521R), pDC24ΔNCgl1496-PgapA_thrA(Q58R, S352P, G433R, W521R, N710D), pDC24ΔNCgl1496-PgapA_thrA(S352P, G433R, W521R, N710D), pDC24ΔNCgl1496-PgapA_thrA(Q58R, S352P, G433R, W521R, N710D, Y784C), and pDC24ANCgl1496-PgapA_thrA(WT) constructed in Example 4-1 were each transformed into an O-acetyl homoserine-producing strain KCCM12634P (Korean Patent No. 10-2182497) by electroporation. Transformed strains were obtained from selection media comprising kanamycin (25 mg/L), and were named KCCM12634P ΔNCgl1496 :: PgapA_thrA(S352P, G433R, W521R), KCCM12634P ΔNCgl1496 :: PgapA_thrA(Q58R, S352P, G433R, W521R, N710D), KCCM12634P ΔNCgl1496 :: PgapA_thrA(S352P, G433R, W521R, N710D), KCCM12634P ΔNCgl1496 :: PgapA_thrA(Q58R, S352P, G433R, W521R, N710D, Y784C), and KCCM12634P ΔNCgl1496 :: PgapA_thrA(WT), respectively.

### Example 4-3. Evaluation of O-Acetyl Homoserine-Producing Ability

In order to confirm their O-acetyl homoserine-producing abilities, the strains prepared in Example 4-2 were cultured and evaluated as follows. One loop of strain was inoculated into a 250 mL corner-baffle flask containing 25 mL of a production medium, and shaking cultured at 33°C and 200 rpm for 20 hours.

### <Production Medium (pH 7.2)>

Glucose 30 g, KH₂PO₄ 2 g, Urea 3 g, (NH₄)₂SO₄ 40 g, Peptone 2.5 g, Corn steep liquor (CSL, Sigma) 5 g (10 mL), MgSO₄·7H₂O 0.5 g, CaCO₃ 20 g (based on 1 L of distilled water)

After completion of the culture, the O-acetyl homoserine-producing abilities were measured using HPLC, and the analyzed O-acetyl homoserine concentrations are as shown in Table 8 below.

**[Table 8]**

| Strain Name | O-Acetyl Homoserine (g/L) |
|---|---|
| KCCM12634P | 1.05 |
| KCCM12634PΔNCgl1496::PgapA_thrA(WT) | 1.4 |
| KCCM12634PΔNCgl1496::PgapA_thrA(S352P,G433R,W521R) | 1.8 |
| KCCM12634PΔNCgl1496::PgapA_thrA(Q58R,S352P,G433R,W521R,N 710D) | 1.7 |
| KCCM12634PΔNCgl1496::PgapA_thrA(S352P,G433R,W521R,N710D) | 1.55 |
| KCCM12634PΔNCgl1496::PgapA_thrA(Q58R,S352P,G433R,W521R,N 710D,Y784C) | 1.6 |

As shown in Table 7 above, when a wild-type *thrA* gene derived from *Escherichia coli was* introduced, it was confirmed that O-acetyl homoserine production increased compared to the parent strain KCCM12634P. In addition, when a variant *thrA* gene was introduced, O-acetyl homoserine production was increased by about 111% to about 128% compared to KCCM12634PΔNCgl1496:: PgapA_thrA(WT) into which the wild-type *thrA* gene had been introduced. In particular, O-acetyl homoserine-producing abilities in KCCM12634PΔNCgl1496:: PgapA_thrA(S352P, G433R, W521R), KCCM12634PΔNCgl1496:: PgapA_thrA(Q58R, S352P, G433R, W521R, N710D), KCCM12634PΔNCgl1496::PgapA_thrA(S352P, G433R, W521R, N710D), and KCCM12634PΔNCgl1496::PgapA_thrA(Q58R, S352P, G433R, W521R, N710D, Y784C) greatly increased compared to the parent strain KCCM12634P. From these results, it was found that *thrA* mutation improves the activity of ThrA, thereby enhancing the O-acetyl homoserine-producing ability of the strain.

From the foregoing description, those skilled in the art to which the present disclosure pertains will be able to understand that the present disclosure may be embodied in other specific forms without modifying the technical concepts or essential characteristics thereof. In this regard, the embodiments described above should be understood to be illustrative rather than restrictive in every respect. The scope of the present disclosure should be construed as the meaning and scope of the appended claims rather than the detailed description, and all changes or variations derived from the equivalent concepts fall within the scope of the present disclosure.

## Claims

1. A protein variant, comprising an amino acid sequence wherein, in the amino acid sequence of SEQ ID NO: 12, from the N-terminus, the amino acid corresponding to position 58 is substituted with arginine (R), the amino acid corresponding to position 352 is substituted with proline (P), the amino acid corresponding to position 433 is substituted with arginine (R), the amino acid corresponding to position 521 is substituted with arginine (R), the amino acid corresponding to position 710 is substituted with aspartic acid (D), or the amino acid corresponding to position 784 is substituted with cysteine (C); or a combination thereof.

2. The protein variant according to claim 1, comprising:
an amino acid sequence wherein, from the N-terminus, the amino acid corresponding to position 352 is substituted with proline (P), the amino acid corresponding to position 433 is substituted with arginine (R), and the amino acid corresponding to position 521 is substituted with arginine (R);
an amino acid sequence wherein, from the N-terminus, the amino acid corresponding to position 58 is substituted with arginine (R), the amino acid corresponding to position 352 is substituted with proline (P), the amino acid corresponding to position 433 is substituted with arginine (R), the amino acid corresponding to position 521 is substituted with arginine (R), and the amino acid corresponding to position 710 is substituted with aspartic acid (D);
an amino acid sequence wherein, from the N-terminus, the amino acid corresponding to position 352 is substituted with proline (P), the amino acid corresponding to position 433 is substituted with arginine (R), the amino acid corresponding to position 521 is substituted with arginine (R), and the amino acid corresponding to position 710 is substituted with aspartic acid (D); or
an amino acid sequence wherein, from the N-terminus, the amino acid corresponding to position 58 is substituted with arginine (R), the amino acid corresponding to position 352 is substituted with proline (P), the amino acid corresponding to position 433 is substituted with arginine (R), the amino acid corresponding to position 521 is substituted with arginine (R), the amino acid corresponding to position 710 is substituted with aspartic acid (D), and the amino acid corresponding to position 784 is substituted with cysteine (C).

3. The protein variant according to claim 1, wherein the protein variant has at least 90% identity to the amino acid sequence of SEQ ID NO: 12.

4. The protein variant according to claim 1, wherein the protein variant comprises the amino acid sequence of SEQ ID NO: 15, SEQ ID NO: 16, SEQ ID NO: 17, or SEQ ID NO: 18.

5. A polynucleotide encoding the protein variant according to claim 1.

6. A microorganism of the genus *Corynebacterium,* comprising at least one selected from the group consisting of the protein variant according to claim 1 and a polynucleotide encoding the protein variant.

7. The microorganism according to claim 6, wherein the microorganism of the genus *Corynebacterium* is *Corynebacterium glutamicum*.

8. The microorganism according to claim 6, wherein the microorganism has an increased L-amino acid-producing ability compared to a microorganism of the genus *Corynebacterium* not comprising at least one selected from the group consisting of the protein variant and a polynucleotide encoding the protein variant.

9. A method for producing an L-amino acid, comprising culturing the microorganism according to claim 6 in a medium.

10. The method according to claim 9, further comprising recovering an L-amino acid from a cultured medium or a cultured microorganism.

11. The method according to claim 9, wherein the L-amino acid is selected from the group consisting of L-threonine, L-isoleucine, O-acetyl L-homoserine, and L-glycine.

12. Use of a microorganism for producing an L-amino acid, wherein the microorganism comprises the protein variant according to any one of claims 1 to 4; a polynucleotide encoding the protein variant; or at least one selected from the group consisting of the protein variant and a polynucleotide encoding the protein variant.
